# FASCICULE DE BREVET EUROPEEN

(11) **EP 0 360 127 B1**
(45) Date de publication et mention de la délivrance du brevet: **07.12.1994**
(21) Numéro de dépôt: 89116817.1
(22) Date de dépôt: 12.09.1989
(51) Int. Cl.: C07C 49/447, C07C 45/62, C07C 69/14, C11B 9/00, A61K 7/46

(54) **Dérivés oxygénés bicycliques et leur utilisation à titre d'ingrédients parfumants**
Bicyclische sauerstoffhaltige Derivate und ihre Verwendung als parfümierende Bestandteile
Oxygenated bicyclic derivatives and their use as perfuming ingredients

(30) Priorité: 23.09.1988 CH 3554/88
(43) Date de publication de la demande: 28.03.1990
(73) Titulaire: FIRMENICH SA, CH-1211 Genève 8 (CH)
(72) Inventeur: Naef, Ferdinand, CH-1227 Carouge (CH); Vial, Christian, CH-1219 Le Lignon (CH)
(74) Mandataire: Salvadori, Giuseppe

(56) Documents cités:
- DE-A- 2 327 417
- FR-A- 2 203 641
- TETRAHEDRON. vol. 23, 1967, pages 1259-1265, Pergamon Press Ltd, IR; R.K. MATHUR et al.: "Terpenoids-XCVIII. Synthesis of (+/-)4-dimethyldihydroeudesmol and (+/-)trans-5alpha,9beta-dimethyl-2-decalone"

## Description

La présente invention concerne des dérivés oxygénés de la décaline, leur utilisation comme ingrédients odoriférants dans la préparation de parfums et de produits parfumés et un procédé de préparation desdits dérivés de la décaline.

Les dérivés de la présente invention répondent à la formule générale 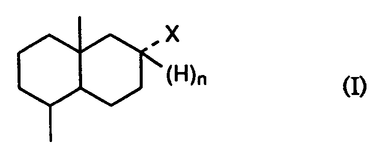
dans laquelle la ligne pointillée peut désigner une liaison simple ou double, l'indice n vaut zéro ou 1 et X sert à définir un groupe -O-acyle inférieur (n=1) ou l'oxygène (n=0).

Comme groupe acyle inférieur on entend un radical C₁ à C₆, de préférence saturé linéaire ou ramifié, par exemple le formyle, l'acétyle, le propionyle ou le butyryle. Il représente de préférence un groupe formyle ou acétyle. C'est ainsi que la formule (I) sert à désigner aussi bien des esters, tels le perhydro-5,8a-diméthyl-2-naphtyl formiate ou perhydro-5,8a-diméthyl-2-naphtyl acétate, que la cétone correspondante, ou perhydro-5,8a,diméthyl-2-naphtalénone.

Les composés (I) sont des entités chimiques nouvelles, à l'exception de la perhydro-5α,8aβ-diméthyl-2-trans-naphtalénone dont la synthèse est décrite par R. K. Mathur et A. S. Rao dans Tetrahedron 23, 1259 (1967) sans qu'il y soit décrite, ni suggérée, aucune propriété particulière de cette naphtalénone du point de vue olfactif.

Le brevet FR-B 1'593'814 décrit des composés décaliniques de formule 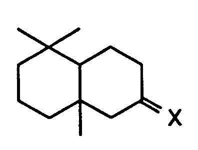
dans laquelle X représente soit l'oxygène, soit deux radicaux monovalents, l'un de ces radicaux étant l'hydrogène et l'autre un reste -O-CO-R dans lequel R représente l'hydrogène ou un groupe hydrocarboné aliphatique, saturé, linéaire ou ramifié de 1 à 6 atomes de carbone ou encore un groupe hydrocarboné aliphatique non saturé, linéaire ou ramifié de 2 à 6 atomes de carbone. Lesdits composés sont dotés de propriétés odorantes utiles et ont trouvé un emploi apprécié en parfumerie où ils contribuent à développer une note boisée et ambrée de grande distinction et élégance dans bon nombre de compositions.

Nous avons maintenant découvert que les composés représentés par la série de leurs homologues inférieurs de formule (I) sert à conférer, modifier ou renforcer les propriétés olfactives de parfums, bases parfumées et produits parfumés dans lesquels ils sont incorporés. Tout en développant une note odorante de type boisé, comme leurs homologues supérieurs mentionnés dans l'antériorité citée, ils possèdent des caractères distincts qui rendent leur utilisation nouvelle et originale. Ils servent en effet à développer des notes odorantes boisées, vertes, terreuses qui rappellent, pour certains d'entre eux, l'odeur d'iris ou celle plus particulière du bois de cèdre. Les proportions dans lesquelles les composés de l'invention peuvent être employés pour produire les effets désirés varient dans une gamme assez étendue. Des concentrations de l'ordre de 2 à 10, voire 20% en poids par rapport au poids total de la composition dans laquelle ils sont incorporés peuvent être parfaitement indiquées pour atteindre les effets recherchés. L'homme de l'art sait par expérience que ces valeurs de concentration dépendent non seulement de l'effet spécifique souhaité, mais également de la nature du produit que l'on désire parfumer et de celle des coingrédients dans une composition donnée. Par ailleurs, de telles valeurs peuvent être inférieures à celles indiquées ci-dessus lorsque les composés de l'invention sont utilisés pour le parfumage de produits tels les savons, les cosmétiques, les détergents ou lorsqu'ils sont incorporés à des supports variés, par exemple minéraux ou polymériques pour la manufacture des désodorisants d'air ambiant. Dans ces cas, leur concentration peut être de l'ordre de 0,2 à 1,0% en poids par rapport au poids total du produit ainsi parfumé.

Les exemples spécifiques indiqués ci-après donnent un aperçu des possibilités d'utilisation des composés (I). Bien entendu l'emploi de ces composés ne saurait y être limité. Les composés de l'invention s'accordent en effet avec la plupart des coingrédients parfumants usuels ainsi qu'avec les supports couramment utilisés en parfumerie. Comme indiqué plus haut, ils servent à parfumer une grande variété de produits de consommation dont les savons, les cosmétiques, les détergents, les adoucissants, les produits d'entretien ou les désodorisants aussi bien corporels que d'ambiance.

Le composés de formule (I) sont des entités chimiques nouvelles, à l'exeption de la (±)-perhydro-5α,8aβ-diméthyl-2-trans-naphthalénone. Ils sont préparés à l'aide d'un procédé original, procédé qui constitue également un objet de la présente invention. Ledit procédé est caractérisé par
a. une hydrogénation catalytique d'une cétone décalinique de formule 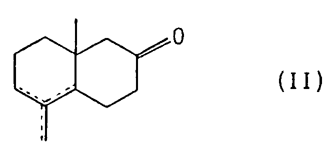 contenant une double liaison dans l'une des positions indiquées par les pointillés, pour fournir la cétone de formule 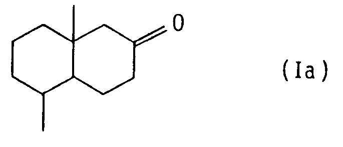
b. suivie d'une hydrogénation catalytique de ladite cétone (Ia) pour donner le carbinol de formule 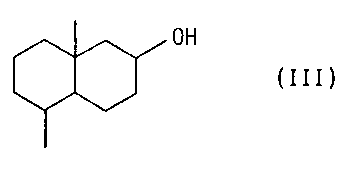 et
c. de l'estérification dudit carbinol pour fournir l'ester de formule 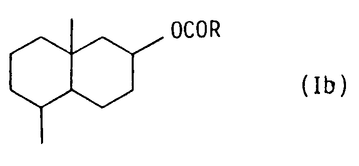 dans laquelle R sert à définir un atome d'hydrogène ou un radical monovalent alkyle de C₁ à C₆ linéaire ou ramifié.

La cétone de formule (II), utilisée comme produit de départ dans le procédé décrit ci-dessus, peut être obtenue suivant un procédé analogue à un procédé connu, notamment décrit par C.R. Bennett et R.C. Cambie dans Tetrahedron 23, 927 (1967).

La cétone (II) peut également être préparée suivant Marshall et al. [voir J. Am. Chem. Soc. 88, 3408 (1966)].

L'ensemble du procédé défini plus haut peut être illustré à l'aide du schéma que voici : 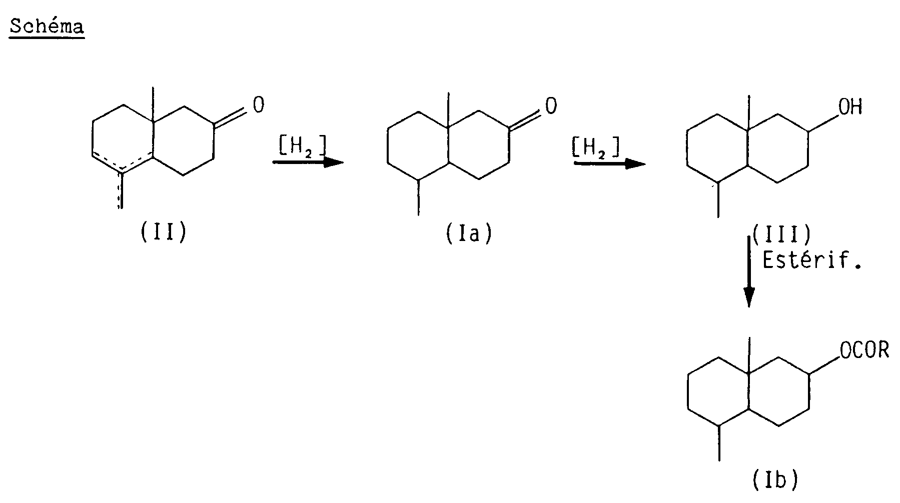
Compte tenu de la présence dans leur molécule de plusieurs centres d'asymétrie, les composés de formule (I) peuvent se présenter sous forme de dérivés racémiques ou d'isomères de configuration optiquement actifs. Quoique l'odeur de ces différents composés appartient à une même famille olfactive, leur caractère propre peut varier en fonction de la structure de chaque composé. Pour toute utilisation pratique selon l'invention, les composés (I) peuvent être employés soit sous forme d'un mélange d'isomères, soit sous la forme d'un énantiomère particulier.

Les composés (I), sous leur forme optiquement active, peuvent être obtenus grâce au procédé décrit plus haut (voir Schéma) à partir des cétones (II), eux-mêmes obtenus à partir d'acide podocarpique ou du cétoester de formule 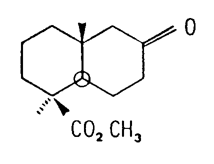
suivant la méthode décrite par R.A. Bell et M.B. Gravestock, Can. J. Chem. 48, 1105 (1970).

L'hydrogénation catalytique qui caractérise les étapes a. et b. du procédé de l'invention décrit plus haut peut être effectuée au moyen de catalyseurs usuels connus pour promouvoir la réduction d'une double liaison cyclohexénique, respectivement de la fonction cétonique. Ainsi des catalyseurs constitués par un métal tel le platine, le palladium ou le nickel, ou un oxyde tel le PtO₂ peuvent parfaitement convenir pour ces opérations.

Des exemples spécifiques de préparation et d'utilisation des composés de l'invention sont donnés ci-après. Les températures sont indiquées en degrés centigrades et les abréviations ont le sens usuel dans l'art.

### Exemple 1

### Préparation de (-)-(4aS)-perhydro-5 bêta,8a bêta-diméthyl-2-trans-naphtalénone

0,89 g (5 mMole) d'un mélange de cétones décaliniques insaturées de formule (II), obtenu comme indiqué ci-après, ont été hydrogénés sous pression atmosphérique dans 25 ml d'acide acétique glacial et en présence de 50 mg de PtO₂. Après absorption de la quantité théorique d'hydrogène (env. 2 h à température ambiante), le mélange réactionnel a été filtré, concentré, repris à l'éther. La phase éthérée a été lavée avec du NaHCO₃, puis avec de l'eau jusqu'à neutralité et enfin séchée sur du MgSO₄, concentré et distillé dans un four à boules [Eb. 95°/0,9 mmHg]. 0,90 g de la cétone désirée ont été ainsi obtenus sous forme cristallisée. La recristallisation dans l'alcool éthylique a fourni un échantillon analytique ayant F. = 67-70° ; [alpha]²⁰_{D} = -51,4° (CH₃OH).
- SM :: M⁺ = 180(27) ; m/e : 162(15) ; 147(31) ; 138(24) ; 122(67) ; 109(77) ; 95(100) ; 81(73) ; 67(68) ; 55(64) ; 41(61).
- RMN (360 MH_{z} ; CDCl₃) :: 0,89(3H,s) ; 0,93(3H,d,J=7 cps) ; 1,98(1H,dd, J₁=13, J₂=2 cps) ; 2,15(1H,d,J=13 cps) ; 2,33(1H,ddd,J₁=J₂=12 ; J₃=7 cps) ; 2,43(dddd,J₁=12 ; J₂=5,5 ; J₃=J₄=2 cps) delta ppm.
Le produit obtenu possède une odeur boisée assez discrète et agréable avec des nuances d'iris et un caractère un peu camphré.
Le mélange des cétones décaliniques de formule (II), utilisé comme produit de départ dans le procédé décrit ci-dessus, a été obtenu à partir de l'acide (4aR)-perhydro-1 alpha,4a bêta-diméthyl-6-oxo-1-trans-naphtalène carboxylique par oxydation à l'aide de tétracétate de plomb selon le procédé décrit par Bennett et Cambie [Tetrahedron 23, 927 (1967)]. Ledit acide naphtalène carboxylique a été préparé à son tour à partir de l'hydroperoxyde obtenu à partir d'acide podocarpique suivant Austr. J. Chem. 35, 183 (1982) ou à partir de (4aR)-perhydro-1 alpha,4a bêta-diméthyl-6-oxo-1-trans-naphtalène carboxylate de méthyle [voir Can. J. Chem. 48, 1105 (1970)]. Le mélange des cétones obtenu était constitué à raison de 52 : 17 : 31 parties par les cétones de structure 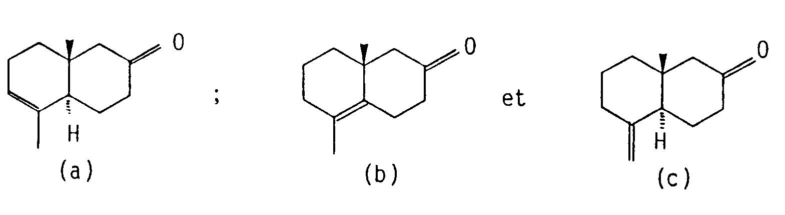
respectivement.
- RMN (360 MH_{z}, CDCl₃) :: (a) : 0,78(3H,s) ; 5,41(1H,m) delta ppm ;
(b) : 1,04(3H,s) delta ppm
(c) : 0,72(3H,s) ; 4,52 et 4,83(1H chacun, 2 m) delta ppm.

Le mélange a été utilisé pour l'étape suivante.

### Exemple 2

### Préparation de l'acétate de (+)-(2S)-perhydro-5 bêta,8a bêta-diméthyl-2 bêta-trans-naphtyle

a. La cétone obtenue comme décrit à l'Exemple 1, a été soumise à une hydrogénation dans le méthanol en présence de palladium sous une pression de 15 bar (durée : 20 h, temp. : 70°). Le mélange réactionnel a été refroidi et filtré, puis concentré et repris à l'éther. La phase éthérée a été séchée et concentrée. Une distillation dans un four à boules à fourni à une température du four de 105°, 230 mg de (+)-(2S)-perhydro-5 bêta, 8a bêta-diméthyl-2 bêta-trans-naphtalénol ayant F = 68-71° et [alpha]²⁰_{D} = +11,4°(CH₃OH) ;
   - SM :: M⁺ = 182(6) ; m/e : 164(25) ; 149(100) ; 135(32) ; 121(35) ; 108(58) ; 95(76) ; 81(75) ; 67(69) ; 55(62) ; 41(61).
   - RMN (360 MH_{z}, CDCl₃) :: 0,91(3H,d,J=7 cps) ; 1,14(3H,s) ; 4,11(1H,m) delta ppm.
b. Un mélange constitué par 265 mg (1,45 mMole) du carbinol obtenu sous lettre a. ci-dessus, 180 mg d'anhydride acétique (1,76 mMole) et 10 ul d'acide sulfurique concentré a été maintenu sous agitation pendant 1 h à 40°, puis il a été dilué à l'eau et extrait à l'éther. Les extraits organiques combinés ont été lavés avec du NaHCO₃, de l'eau jusqu'à neutralité, séchés, concentrés et distillés à l'aide d'un four à boules [Eb. 105°/0,5 mmHg]. 276,7 mg de l'acétate désiré ont été ainsi obtenus. Par chromatographie sur colonne remplie de gel de silice avec hexane/diéthyl éther comme éluant, on a obtenu 255,8 mg de l'acétate pur ; [alpha]²⁰_{D} = +9,25° (CH₃OH).
   - SM :: M⁺ = 224(0) ; m/e : 164(54), 149(72), 135(80), 121(43), 108(87), 95(59), 51(55), 67(53), 55(50), 43(100) ;
   - RMN (360 MH_{z}, CDCl₃) :: 0,92(3H,d,J=7 cps) ; 1,04(3H,s) ; 2,04(3H,s) ; 5,03(1H,m) delta ppm.

Le produit obtenu possède une note boisée distincte, vaguement ambrée et poudrée ; possède une nuance verte.

### Exemple 3

### Préparation de (±)-perhydro-5,8a-diméthyl-2-trans-naphtalénone

Un mélange de 500 mg (2,8 mMole) de 3,4,6,7,8,8a-hexahydro-5,8a-diméthyl-2(1H)-naphtalénone [préparée suivant Marshall et al., J. Am. Chem. Soc. 88, 3408 (1966)], 20 ml d'acide acétique glacial et 50 mg de PtO₂ a été hydrogéné dans les conditions définies à l'Exemple 1. L'hydrogénation toutefois a été poursuivie pendant 48 h au lieu de 2 h. Après purification sur colonne de gel de silice avec un mélange 9 : 1 d'hexane/diéthyl éther comme éluant, on a obtenu 171,7 mg de la cétone désirée dont les données analytiques étaient en tous points identiques à celle du produit obtenu conformément à l'Exemple 1. La cétone était accompagnée de 250 mg de perhydro-5,8a-diméthyl-2-trans-naphtalénol.

### Exemple 4

### Préparation de (±)-acétate de perhydro-5,8a-diméthyl-2-trans-naphtyle

Suivant le procédé décrit à l'Exemple 2, on a acétylé 250 mg (1,37 mMole) de perhydro-5,8a-diméthyl-2-trans-naphtalénol. Par distillation dans un four à boules, on a obtenu l'acétate désiré ayant Eb. 110°/0,4 mmHg dont les caractères analytiques étaient en tous points identiques à ceux du produit obtenu suivant l'Exemple 2. Le produit possède une note boisée, avec des nuances fleuries et vertes, légèrement camphrée.

### Exemple 5

### Composition parfumante

On a préparé une composition parfumante de base en mélangeant les ingrédients suivants (parties en poids) :

| | |
|---|---|
| Essence de bergamote synth. | 140 |
| Essence de galbanum à 10% * | 80 |
| p-tert-Butyl-cyclohexylacétaldéhyde | 80 |
| Ald. méthyl-octyl acétique à 10% * | 80 |
| Essence de jasmin synth. | 60 |
| Essence de citron. | 60 |
| Mousse de chêne absolue décolorée 50% * | 60 |
| Lavandin absolue | 40 |
| Essence de clou de girofle | 40 |
| 2,4-Diméthylcyclohex-3-ényl carbaldéhyde à 10% * | 40 |
| alpha-Isométhylionone | 40 |
| Essence de patchouli | 20 |
| Tétrarome orange ¹⁾ | 30 |
| Acétate de styrallyle | 30 |
| Essence de néroli Bigarade | 20 |
| Sandela (marque enregistrée) ²⁾ | 10 |
| Musc DTI ³⁾ | 10 |
| Muscone ⁴⁾ à 10% * | 10 |
| Benjoin résinoïde à 50% * | 20 |
| Labdanum résinoïde à 50% * | 20 |
| Mousse crystal | 10 |
| Total | 9̅0̅0̅ |

| | |
|---|---|
| * dans le dipropylène glycol (DIPG) | |
| 1) essence d'orange déterpénée | |
| 2) origine : L. Givaudan SA, Vernier, Suisse | |
| 3) acétyl diméthyl butyl indane ; Firmenich SA, Genève, Suisse | |
| 4) Firmenich SA, Genève, Suisse | |

A l'aide de la base ainsi obtenue, on a ensuite préparé les compositions suivantes (parties en poids) :

| | Base | DIPG | comp. 1 ¹⁾ | comp. 2 ²⁾ |
|---|---|---|---|---|
| A | 90 | 10 | -- | -- |
| B | 90 | -- | 10 | -- |
| C | 90 | -- | -- | 10 |

| | | | | |
|---|---|---|---|---|
| 1) composé selon l'Exemple 1 | | | | |
| 2) composé selon l'Exemple 2 | | | | |

La composition A représente une composition de type Chypre, boisée, lavande, hespéridée dont les caractères olfactifs sont peu équilibrés.

La composition B a par contre acquis un équilible plaisant grâce à l'addition du composé 1 ; elle est plus boisée que la composition A et la note hespéridée est harmonieusement intégrée dans l'ensemble.

La composition C a également un caractère plus arrondi que la composition A. Elle est plus boisée et montre également une nuance ambrée-aromatique.

## Revendications

1. Cétones ou esters décaliniques de formule 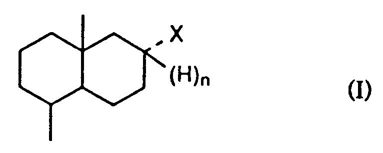 dans laquelle la ligne pointillée peut désigner une liaison simple ou double, l'indice n vaut zéro ou 1 et X sert à définir un groupe -O-acyle inférieur (n=1) ou l'oxygène (n=0), à l'exclusion de la (±) -perhydro-5α, 8a β -diméthyl-2-trans-naphtalénone.

2. Esters décaliniques selon la revendication 1 définis par la formule suivante 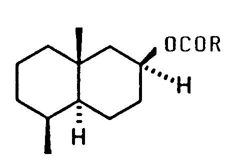 dans laquelle R sert à désigner un atome d'hydrogène ou un radical monovalent alkyle de C₁ à C₆ linéaire ou ramifié.

3. En tant que composé selon la revendication 1, la (-)-(4aS)-perhydro-5β,8aβ-diméthyl-2-trans-naphtalénone.

4. En tant que composé selon la revendication 1, l'acétate de (+)-(2S)-perhydro-5β,8aβ-diméthyl-2β-trans-naphtayle.

5. Procédé pour la préparation des cétones ou esters décaliniques selon la revendication 1, caractérisé en ce qu'on
a. soumet une cétone décalinique de formule 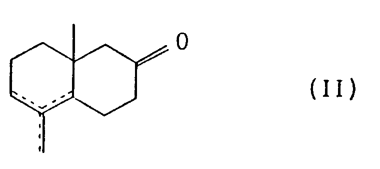 contenant une double liaison dans l'une des positions indiquées par les pointillés, à une hydrogénation catalytique pour fournir une cétone de formule 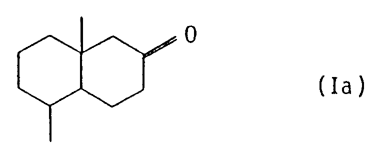
b. soumet ladite cétone de formule (Ia) à une hydrogénation catalytique pour donner le carbinol de formule 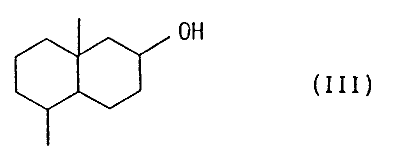 et
c. esterifie ledit carbinol pour fournir l'ester de formule 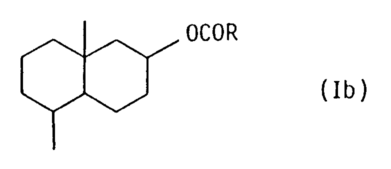 dans laquelle R sert à définir un atome d'hydrogène ou un radical monovalent alkyle de C₁ à C₆ linéaire ou ramifié.

6. Procédé selon la revendication 5 caractérisé en ce que l'hydrogénation catalytique suivant l'étape a. du procédé s'effectue en présence de palladium, de platine ou de PtO₂.

7. Procédé selon la revendication 5 caractérisé en ce que l'hydrogénation catalytique suivant l'étape b. du procédé s'effectue en présence de palladium.

8. Utilisation à titre d'ingrédient parfumant d'une cétone ou d'un ester décalinique de formule 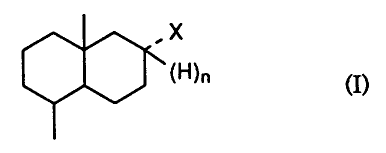 dans laquelle la ligne pointillée peut désigner une liaison simple ou double, l'indice n vaut zéro ou 1 et X sert à définir un groupe -O-acyle inférieur (n=1) ou l'oxygène (n=0).

9. Produits parfumés contenant à titre d'ingrédient actif l'un des composés selon la revendication 8.

10. A titre de produit parfumé selon la revendication 9, un savon, un détergent, un adoucissant textile ou un désodorisant corporel ou d'air ambiant.

11. Composition parfumante contenant à titre d'ingrédient actif l'un des composés selon la revendication 8.

## Claims

1. Ketones or decalin esters of formula 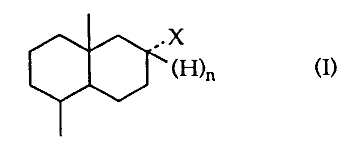 wherein the dotted line can designate a single or double bond, index n takes the value 0 or 1 and X defines a lower O-acyl radical (n=1) or an oxygen atom (n=0), provided that (±)-perhydro-5α,8aβ-dimethyl-2-trans-naphthalenone is excluded.

2. Decalin esters according to claim 1 represented by the following formula 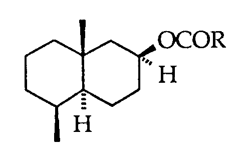 wherein R designates a hydrogen atom or a monovalent linear or branched alkyl radical having from 1 to 6 carbon atoms.

3. As a compound according to claim 1, (-)-(4aS)-perhydro-5β,8aβ-dimethyl-2-trans-naphthalenone.

4. As a compound according to claim 1, (+)-(2S)-perhydro-5β,8aβ-dimethyl-2β-trans-naphthyl acetate.

5. A process for the preparation of decalin ketones or esters according to claim 1, characterized in that
a. a decalin ketone of formula 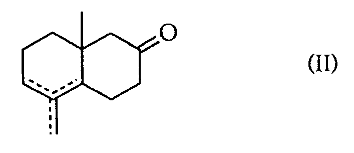 having a double bond in one of the positions indicated by the dotted line, is subjected to a catalytic hydrogenation to give a ketone of formula 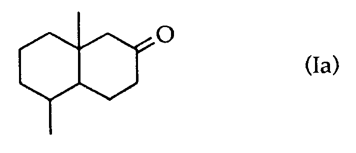
b. the said ketone of formula (Ia) is subjected to a catalytic hydrogenation to give a carbinol of formula 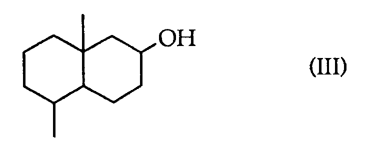 and
c. the said carbinol is esterified to give an ester of formula 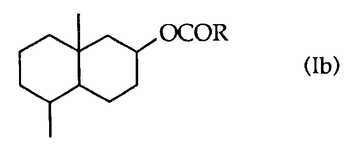 wherein R stands for a hydrogen atom or a monovalent C₁ to C₆ alkyl radical, linear or branched.

6. A process according to claim 5, characterized in that the catalytic hydrogenation according to step a. of said process is carried out in the presence of palladium, platinum or PtO₂.

7. A process according to claim 5, characterized in that the catalytic hydrogenation according to step b. of the said process is carried out in the presence of palladium.

8. Use as a perfuming ingredient of a decalin ketone or ester of formula 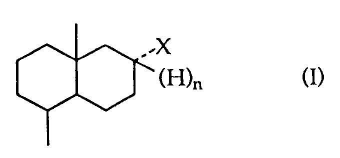 wherein the dotted line can designate a single or double bond, index n takes the value 0 or 1 and X defines a lower O-acyl radical (n=1) or an oxygen atom (n=0).

9. Perfumed products containing as an active ingredient a compound according to claim 8.

10. As a perfumed product according to claim 9, a soap, a detergent, a fabric softener, a body deodorant or a deodorant of ambient air.

11. A perfuming composition containing as an active ingredient a compound according to claim 8.

## Patentansprüche

1. Ketone oder Decalinester der Formel 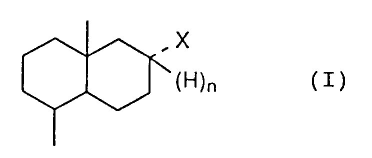 worin die punktierte Linie eine einfache Bindung oder eine Doppelbindung bezeichnen kann, der Index n für 0 oder 1 steht und X zur Definition einer niederen O-Acylgruppe (n = 1) oder Sauerstoff (n = 0) dient, mit Ausnahme des (+/-)-Perhydro-5α,8aβ-dimethyl-2-trans-naphthalinons.

2. Decalinester gemäss Anspruch 1, definiert durch die nachfolgende Formel 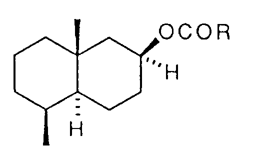 worin R zur Bezeichnung eines Wasserstoffatoms oder eines einwertigen, geradekettigen oder verzweigten Alkylrestes mit C₁ bis C₆ dient.

3. (-)-(4aS)-Perhydro-5β,8aβ-dimethyl-2-trans-naphthalinon, als Verbindung gemäss Anspruch 1.

4. (+)-(2S)-Perhydro-5β,8aβ-dimethyl-2β-trans-naphthyl-acetat, als Verbindung gemäss Anspruch 1.

5. Verfahren zur Herstellung von Ketonen oder Decalinestern gemäss Anspruch 1, dadurch gekennzeichnet, dass man
a. ein Decalinketon der Formel 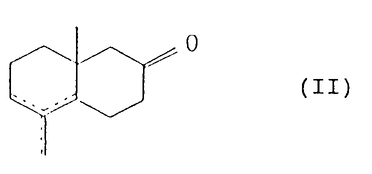 enthaltend eine Doppelbindung in einer der durch die Punktierungen dargestellten Stellungen, einer katalytischen Hydrierung unterwirft, um ein Keton der Formel 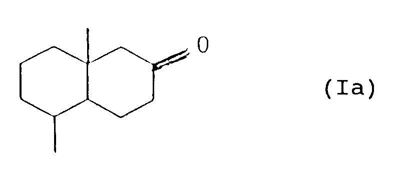 zu erhalten,
b. dieses Keton der Formel (Ia) einer katalytischen Hydrierung unterwirft, um das Carbinol der Formel 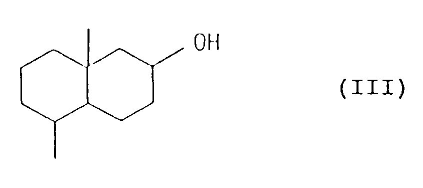 zu erhalten und
c. diese Carbinol verestert, um den Ester der Formel 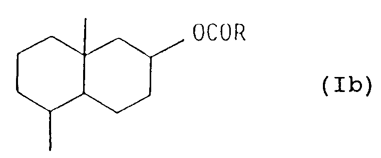 zu erhalten, worin R zur Definition eines Wasserstoffatoms oder eines einwertigen, geradekettigen oder verzweigten Alkylrestes mit C₁ bis C₆ dient.

6. Verfahren gemäss Anspruch 5, dadurch gekennzeichnet, dass die katalytische Hydrierung gemäss der Stufe a. des Verfahrens in Gegenwart von Palladium, Platin oder PtO₂ erfolgt.

7. Verfahren gemäss Anspruch 5, dadurch gekennzeichnet, dass die katalytische Hydrierung gemäss der Stufe b. des Verfahrens in Gegenwart von Palladium erfolgt.

8. Verwendung eines Ketons oder eines Decalinesters der Formel 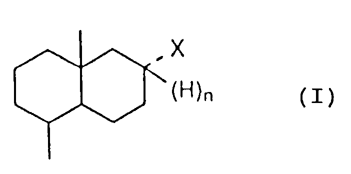 worin die punktierte Linie eine einfache Bindung oder eine Doppelbindung bezeichnen kann, der Index n für 0 oder 1 steht und X zur Definition einer niederen O-Acylgruppe (n = 1) oder Sauerstoff (n = 0) dient, als Riechstoffbestandteil.

9. Parfümierte Artikel enthaltend als aktiven Bestandteil eine der Verbindungen gemäss Anspruch 8.

10. Eine Seife, ein Reinigungsmittel, ein Textilweichmacher oder ein Körper- oder Raum- Desodorant als parfümierter Artikel gemäss Anspruch 9.

11. Riechstoffkompositionen enthaltend als aktiven Bestandteil eine der Verbindungen gemäss Anspruch 8.
